# EUROPÄISCHE PATENTSCHRIFT

(11) **EP 3 973 288 B1**
(45) Veröffentlichungstag und Bekanntmachung des Hinweises auf die Patenterteilung: **10.04.2024**
(21) Anmeldenummer: 20726417.7
(22) Anmeldetag: 18.05.2020
(51) Int. Cl.: G01N 33/49, B01L 3/00, G01N 1/40, C12N 15/10

(54) **MIKROFLUIDISCHES ANALYSESYSTEM ZUR ANALYSE VON BLUTPROBEN**
MICROFLUIDIC ANALYSIS SYSTEM FOR ANALYSING BLOOD SAMPLES
SYSTÈME D'ANALYSE MICROFLUIDIQUE POUR L'ANALYSE D'ÉCHANTILLONS DE SANG

(30) Priorität: 21.05.2019 DE 102019207394
(43) Veröffentlichungstag der Anmeldung: 30.03.2022
(73) Patentinhaber: Robert Bosch GmbH, 70442 Stuttgart (DE)
(72) Erfinder: FRANK, Tino, 6003 Luzern (CH)
(86) Internationale Anmeldenummer: PCT/EP2020/063769
(87) Internationale Veröffentlichungsnummer: WO 2020/234218

(56) Entgegenhaltungen:
- WO-A1-2005/119211
- US-A1- 2014 314 636
- US-B1- 9 700 888

## Beschreibung

Die Erfindung betrifft ein mikrofluidisches Analysesystem zur Analyse von Blutproben sowie ein Verfahren zum Eingeben von Bestandteilen einer Blutprobe in ein entsprechendes mikrofluidisches Analysesystem. Darüber hinaus kann das mikrofluidisches Analysesystem zum Aufnehmen von Bestandteilen einer Blutprobe verwendet werden.

### Stand der Technik

Die in-vitro Diagnostik (IVD) ist ein Feld von Medizinprodukten, welche aus humanen Proben spezifische Größen (z.B. Konzentration eines Moleküls, Vorhandensein einer bestimmten DNA Sequenz, Zusammensetzung von Blut) messen und eine Diagnose und Behandlungsentscheid zulassen. Dies geschieht oft in einer Verkettung von mehreren Laborschritten, wobei die Probe so aufbereitet wird, dass die Zielgröße störungsfrei messbar ist. Dabei werden verschiedene Labormethoden angewandt, die jeweils ein der Methode geeignetes Gerät besitzt.

In point-of-care Geräten ist das Ziel, solche in-vitro Diagnostiktests in einem Gerät abzubilden und die Anzahl manueller Schritte vom Benutzer auf ein Minimum zu reduzieren. Im Idealfall wird das Sample direkt in das Gerät gegeben und der Diagnosetest wird vollautomatisch abgearbeitet. Mikrofluidische Systeme - oft als Lab-on-Chip bezeichnet - eignen sich besonders, um verschiedene biochemische Diagnosemethoden fluidisch zu prozessieren und analysieren. Lab-on-chip Geräte sind in der Regel für eine bestimmte Messmethode (z. B. PCR, Fluoreszenzmessung, ph-Messung) konzipiert.

In der heutigen IVD sind Tests, welche mehre Quantitäten gleichzeitig messen immer mehr gefragt, da die Verknüpfung von mehreren Messgrößen und Anwendung von Datamining salonfähig wird und die Zukunft von IVD Methoden prägen wird. Insbesondere in der Analytik von Genexpressionen sind parallele Messung des Genoms, Transkriptom und des Proteoms gefragt (sogenannte Panomics-Messungen). Dabei wird eine Probe oft nach Volumen fraktioniert, anstelle nach Inhalt, d.h. ein Teil wird zur Analyse der DNA, einer für die RNA und der letztere für Proteine eingesetzt. Ist nur wenig Material vorhanden oder wird seltenes Material, z. B. zirkulierende Tumorzellen, untersucht, ist diese Aufspaltung nicht erwünscht, da Information verloren gehen kann. Methoden, welche die gesamte OMICS-Last misst, bieten für solche Verfahren die beste Ausgangslage, leider existieren nur wenige davon, wegen der herausfordernden und anspruchsvollen Probenvorbereitung.

Die US 2014/314636 A1 offenbart einen Mikrochip, der einen Flüssigkeitskreislauf enthält, der aus einem im Inneren gebildeten Raum besteht und eine im Flüssigkeitskreislauf vorhandene Flüssigkeit veranlasst, sich durch Anwendung der Zentrifugalkraft innerhalb des Flüssigkeitskreislaufs zu bewegen.

Die WO 2005/119211 A1 offenbart eine Vorrichtung zur Aufnahme von Blut und Abtrennung von Blutplasma als Probenflüssigkeit mit einem die Probenflüssigkeit durch Kapillarkräfte aufnehmenden Kanal.

Die US 9 700 888 B1 offenbart eine mikrofluidische Kartusche, die DNA-Tests in Laborqualität und andere amplifikationsbasierte Tests am Point of Care durchführen kann.

### Offenbarung der Erfindung

Hier vorgeschlagen wird ein mikrofluidisches Analysesystem zur Analyse von Blutproben gemäß Anspruch 1.

Das Analysesystem erlaubt in vorteilhafter Weise eine selektive Lyse einer Blutprobe am Point-of-Care in einem geeigneten Gefäß, insbesondere der Probeneingabekammer, über welche die gesamte Probe schrittweise und verlustfrei in ihre Bestandteile auf eine Lab-on-Chip-Plattform überführbar ist. Insbesondere trägt das Analysesystem in besonders vorteilhafter Weise zum selektiven Lysieren von immobilisierten Blutzellen in einem externen Lysetool in einem kleinen Volumen in Kombination mit einer mikrofluidischen Polyprobenkammer bei, welche einen Probeneinzug in einem Zweiphasensystem ermöglicht. Dabei wird die Polyprobenkammer insbesondere durch die Probeneingabekammer gebildet. Weiterhin wird in der Regel die Probenextraktion nicht in Teilproben unterteilt, sondern die Extraktionen finden über die ganze Probemenge statt. Das Analysesystem stellt insbesondere ein Probenaufbereitungssystem dar.

Der Begriff "mikrofluidisch" bezieht sich hier vor allem auf die Größenordnung des mikrofluidischen Analysesystems. Mikrofluidische Systeme sind in der Regel dadurch gekennzeichnet, dass in den darin angeordneten fluidischen Kanälen und Kammern physikalische Phänomene relevant sind, die im Allgemeinen der Mikrotechnik zugeordnet werden. Hierzu zählen beispielsweise Kapillareffekte, Effekte (insbesondere mechanische Effekte) die im Zusammenhang mit Oberflächenspannungen des Fluids stehen. Hinzu zählen weiterhin Effekte wie Thermophorese und Elektrophorese. Diese Phänomene sind in der Mikrofluidik üblicherweise dominant gegenüber Effekten wie der Schwerkraft. Der Begriff "mikrofluidisch" kann auch über die Querschnitte innerhalb der Vorrichtung charakterisiert werden, welche zur Führung des Fluids dienen. Üblich sind beispielsweise Querschnitte im Bereich von 100 µm [Mikrometer] mal 100 µm bis hin zu 800 µm mal 800 µm. Auch deutlich kleinere Querschnitte, beispielsweise im Bereich von 1 µm bis 20 µm [Mikrometer], insbesondere im Bereich von 3 µm bis 10 µm sind möglich.

Bei dem mikrofluidischen Analysesystem handelt es sich insbesondere um ein System für einen (mikrofluidischen) Analyseapparat zur Analyse einer Probe bzw. um ein System, das (zu Analysezwecken) mit einem Analyseapparat (insbesondere einer Analyseeinrichtung eines Analyseapparats) verbunden werden kann. In diesem Zusammenhang bilden das Analysesystem und/oder die Probeneingabekammer insbesondere ein sogenanntes World-to-chip Interface für eine insbesondere universelle LoC-(Lab-on-Chip-)Plattform. Dies bedeutet mit anderen Worte insbesondere, dass es sich bei dem Analysesystem insbesondere um ein (wechselbares) Eingabesystem handeln kann, welches mit einem Analysegerät verbunden werden kann, beispielsweise um eine ggf. in dem Eingabesystem vorprozessierte (aufbereitete) Probe in das Analysegerät einzubringen. Vorzugsweise weist das Analysesystem eine (universelle bzw. standardisierte) Schnittstelle auf. Diese Schnittstelle ist insbesondere derart eingerichtet, dass sie mit einer Probenschnittstelle eines Analyseapparats (insbesondere einer Analyseeinrichtung eines Analyseapparats) korrespondiert. In der Schnittstelle des Analysesystems können beispielsweise die Anschlussleitungen des Analysesystems münden.

Weiterhin kann das mikrofluidische Analysesystem als eine Einheit gebildet sein. Dies bedeutet mit anderen Worten insbesondere, dass zumindest die Probeneingabekammer und die zwei Anschlussleitungen integral bzw. einstückig gefertigt sein können. Das Analysesystem kann hierzu beispielsweise gegossen oder schichtweise aufgebaut, insbesondere dreidimensional gedruckt sein. Als Material für das mikrofluidische Analysesystem wird vorzugsweise ein nichtluftdurchlässiges Polymer vorgeschlagen, z. B. Polycarbonat.

Vorzugsweise sind das mikrofluidische Analysesystem und/oder die Probeneingabekammer probenspezifisch gebildet. Dies bedeutet mit anderen Worten insbesondere, dass das mikrofluidische Analysesystem und/oder die Probeneingabekammer gezielt für einen Einsatzzweck angepasst bzw. modifiziert sind. Dies erlaubt in vorteilhafter Weise, dass zum Analysieren verschiedener Proben lediglich das hier beschriebene Analysesystem gewechselt werden muss, nicht jedoch der gesamte Analyseapparat. Dies trägt zu einem besonders vorteilhaften Einsatz, insbesondere am sog. "Point-of-Care" bei.

Nach einer vorteilhaften Ausgestaltung wird vorgeschlagen, dass die Eingabebereiche jeweils einen Eingaberaum und eine Zugangsöffnung aufweisen, wobei die Bestandteile der Blutprobe über die Zugangsöffnungen in die Eingaberäume der Eingabebereiche eingebbar sind.

Insbesondere sind die (drei) Zugangsöffnungen in derselben (im Wesentlichen horizontalen) Ebene angeordnet. Dies bedeutet mit anderen Worten insbesondere, dass die (drei) Zugangsöffnungen in derselben (im Wesentlichen horizontalen) Querschnittsebene liegen. Darüber hinaus sind die Zugangsöffnungen in diesem Zusammenhang in der Regel im Wesentlichen vertikal ausgerichtet.

In diesem Zusammenhang ist es weiterhin bevorzugt, wenn die Zugangsöffnungen (wenn der ggf. existierende Deckel abgenommen ist) frei zugänglich, insbesondere von oben her frei zugänglich sind. Dies erlaubt in vorteilhafter Weise, dass die Eingaberäume möglichst einfach und schnell mit beispielsweise einer Trennpipette (zumindest teilweise) befüllt werden können.

Die freie Zugänglichkeit kann beispielsweise dadurch erreicht werden, dass die oberhalb der Zugangsöffnungen liegenden (horizontalen) Querschnitte durch die Probeneingabekammer entsprechend gestaltete Aussparungen und/oder eine gemeinsame (entsprechend groß gestaltete) Aussparung vorsehen. In diesem Zusammenhang ist jedoch darauf hinzuweisen, dass ggf. Filter oder Siebe in oder oberhalb der Zugangsöffnungen vorgesehen sein können. In diesem Falle sollte die freie Zugänglichkeit zumindest bis hin zu dem Filter oder Sieb möglich sein. Auch kann vorgesehen sein, dass entsprechende Filter oder Siebe die freie Zugänglichkeit zu den Zugangsöffnungen im hier maßgeblichen Sinne nicht einschränkten.

Nach einer weiteren vorteilhaften Ausgestaltung wird vorgeschlagen, dass für die Probeneingabekammer ein Deckel existiert, mit welchem die Zugangsöffnungen der Eingabebereiche verschließbar sind.

Vorzugsweise ist der Deckel in diesem Zusammenhang so gestaltet, dass er alle drei Zugangsöffnungen gleichzeitig verschließen kann. Dies kann mit anderen Worten auch als ein gemeinsamer Deckel bezeichnet werden. Der Deckel verfügt zudem insbesondere über ein Dichtfunktion, um einen nach außen möglichst abgedichteten Fluidfluss innerhalb des Analysesystems zu ermöglichen.

Erfindungsgemäß weist die Probeneingabekammer mindestens zwei Anschlussleitungen auf, über welche die Bestandteile der Blutprobe in Analysekammern abtransportierbar sind und über welche die Probeneingabekammer mit einem Förderfluid beaufschlagbar ist.

In diesem Zusammenhang können die Anschlussleitungen zum Beispiel in der Art von Kanälen gebildet sein, die an einem ihrer jeweiligen Enden in die Probeneingabekammer münden. An dem anderen ihrer jeweiligen Enden können die Kanüle zum Beispiel in besagte Analysekammern oder Reservoire für das Förderfluid münden.

Ferner weist das mikrofluidische Analysesystem mindestens ein Reservoir auf, welches mit einem Förderfluid gefüllt ist, das über eine erste Anschlussleitung in die Probeneingabekammer gefördert werden kann, um Bestandteile der Blutprobe aus der Probeneingabekammer abzustransportieren.

Nach einer weiteren vorteilhaften Ausgestaltung wird vorgeschlagen, dass die drei räumlich voneinander getrennten Eingabebereiche entlang eines Strömungspfades von einer ersten Anschlussleitung an die Probeneingabekammer zu einer zweiten Anschlussleitung an die Probeneingabekammer hintereinander angeordnet sind. Dies kann mit anderen Worten auch so beschrieben werden, dass die drei räumlich voneinander getrennten Eingabebereiche entlang eines Strömungspfades von einer ersten Anschlussleitung zu einer zweiten Anschlussleitung in Strömungsrichtung aufeinander folgen. Dabei können die Anschlussleitungen an einander gegenüberliegenden Stellen in die Probeneingabekammer münden.

Gleichwohl können die Eingabebereiche, wenn man einen (im Wesentlichen horizontalen) Querschnitt durch die Probeneingabekammer betrachtet, nebeneinander angeordnet sein. Dies bedeutet mit anderen Worten insbesondere, dass die Eingabebereiche in einer Querschnittsebene nebeneinander und entlang eines Strömungspfades hintereinander liegen können.

Nach einer weiteren vorteilhaften Ausgestaltung wird vorgeschlagen, dass die Eingabebereiche durch mindestens zwei senkrecht (bezogen auf einen Boden der Probeneingabekammer) ausgerichtete Trennwände voneinander abgegrenzt sind. Dies bedeutet mit anderen Worten insbesondere, dass die Eingabebereiche durch mindestens zwei im Wesentlichen vertikal ausgerichtete Trennwände voneinander abgegrenzt sind.

Die Trennwände können sich in diesem Zusammenhang zumindest teilweise durch die Probeneingabekammer erstrecken. Beispielsweise können sich die Trennwände ausgehend von einem Boden der Probeneingabekammer bis zu einem bestimmten Höhe innerhalb der Probeneingabekammer oder sogar bis hin zu einem oberen Ende der Probeneingabekammer erstrecken. Das obere Ende der Probeneingabekammer kann in diesem Zusammenhang ggf. eine Anlageebene für einen Deckel bilden. Die Trennwände können sich grundsätzlich gleich hoch in die Probeneingabekammer erstrecken. Denkbar sind jedoch auch unterschiedliche Trennwandhöhen.

Nach einer weiteren vorteilhaften Ausgestaltung wird vorgeschlagen, dass jeder Eingabebereich entlang eines Strömungspfades eine Hauptdurchströmungsrichtung von oben nach unten oder von unten nach oben hat, wobei zwei benachbart zueinander angeordnete Eingabebereiche der Probeneingabekammer, entlang des Strömungspfades jeweils unterschiedliche Hauptdurchströmungsrichtungen haben.

Nach einer weiteren vorteilhaften Ausgestaltung wird vorgeschlagen, dass jeder Eingabebereich entlang eines Strömungspfades eine Hauptdurchströmungsrichtung von oben nach unten oder von unten nach oben hat, wobei zwei benachbart zueinander angeordnete Eingabebereiche der Probeneingabekammer, entlang des Strömungspfades gleiche Hauptdurchströmungsrichtungen haben.

Nach einer weiteren vorteilhaften Ausgestaltung wird vorgeschlagen, dass zwischen zwei benachbart zueinander angeordneten Eingabebereichen mit gleicher Hauptdurchströmungsrichtung eine Umlenkstrecke existiert mit welcher der Strömungspfad in der zu der Hauptdurchströmungsrichtung besagter Eingabebereiche entgegengesetzten Richtung umgelenkt ist.

Besagte Umlenkstrecke kann beispielsweise zwischen zwei unmittelbar benachbarten Trennwänden gebildet sein. In diesem Zusammenhang kann vorgesehen sein, dass sich diese zwei Trennwände in senkrechter bzw. vertikaler Richtung nur teilweise überlappen. Beispielsweise kann eine sich von einem Boden aus erstreckende Trennwand kurz vor einer Ebene enden, von der aus sich die benachbarte Trennwand von oben nach unten (aber nicht ganz bis zum Boden) erstreckt.

Nach einer weiteren vorteilhaften Ausgestaltung wird vorgeschlagen, dass mindestens einer der Eingabebereiche einen Eingabefilter aufweist, welcher den Eingabebereich verdeckt. In diesem Zusammenhang kann der Eingabefilter den Eingabebereich zumindest teilweise oder sogar vollständig bedecken bzw. verdecken.

Nach einem weiteren Aspekt wird auch ein Verfahren zum Eingeben von Bestandteilen einer Blutprobe in ein hier beschriebenes mikrofluidisches Analysesystem vorgeschlagen, aufweisend die folgenden Schritte:
a) Zeitlich aufeinanderfolgende und voneinander getrennte Zugabe der Bestandteile der Blutprobe in die Eingabebereiche der Probeneingabekammer, gemäß einer Vorgabe,
b) Mindestens einmalige Zugabe eines Trennfluids in die Eingabebereiche der Probeneingabekammer zur Abtrennung von Bestandteilen der Blutprobe entlang des Strömungspfades von einer ersten Anschlussleitung an die Probeneingabekammer zu einer zweiten Anschlussleitung an die Probeneingabekammer.

Die Schritte a) und b) können in der angegebenen Reihenfolge hintereinander durchgeführt werden. Darüber hinaus können die Schritte a) und b) auch (zeitlich) zumindest teilweise parallel oder sogar gleichzeitig durchgeführt werden.

Die Vorgabe kann beispielsweise dergestalt sein, dass zwischen den Zugaben ein vorbestimmter zeitlicher Abstand einzuhalten ist. Bevorzugt ist die Vorgabe aber dergestalt, dass bestimmte Bestandteile der Blutprobe in bestimmte Eingabebereiche zugegeben werden sollen. Vorzugsweise wird immer nur ein bestimmter Bestandteil der Blutprobe in einen bestimmten der Eingabebereiche eingebracht. Dies kann auch so beschrieben werden, dass die Eingabebereiche blutprobenbestandteilspezifisch sind.

In einem Schritt c) kann sich an die Schritte a) und b) ein Austragen der durch das Trennfluid voneinander getrennten Bestandteile der Blutprobe aus der Probeneingabekammer anschließen. Dabei können die getrennten Bestandteile in ein mit der Probeneingabekammer verbundenes Analysegerät aufgenommen werden. Die Probeneingabekammer kann dabei als Bestandteile eines sog. Lab-on-Chip mit dem Analysegerät verbunden sein.

Nach einem weiteren Aspekt kann auch eine Verwendung eines hier beschriebenen mikrofluidischen Analysesystems zum Aufnehmen von Bestandteilen einer Blutprobe angegeben werden. Dabei kann die Verwendung so erfolgen, dass die Bestandteile der Blutprobe zeitlich aufeinanderfolgend und voneinander getrennt in die Eingabebereiche der Probeneingabekammer (gemäß einer Vorgabe) zugegeben und/oder über die Probeneingabekammer in ein Lab-on-Chip aufgenommen werden.

Die im Zusammenhang mit dem Analysesystem erörterten Details, Merkmale und vorteilhaften Ausgestaltungen können entsprechend auch bei dem hier vorgestellten Verfahren und/oder der Verwendung auftreten und umgekehrt. Insoweit wird auf die dortigen Ausführungen zur näheren Charakterisierung der Merkmale vollumfänglich Bezug genommen.

Die hier vorgestellte Lösung sowie deren technisches Umfeld werden nachfolgend anhand der Figuren näher erläutert. Es ist darauf hinzuweisen, dass die Erfindung durch die gezeigten Ausführungsbeispiele nicht beschränkt werden soll. Insbesondere ist es, soweit nicht explizit anders dargestellt, auch möglich, Teilaspekte der in den Figuren erläuterten Sachverhalte zu extrahieren und mit anderen Bestandteilen und/oder Erkenntnissen aus anderen Figuren und/oder der vorliegenden Beschreibung zu kombinieren. Es zeigen schematisch:
- Fig. 1:: eine beispielhafte Ausführungsform eines hier vorgeschlagenen mikrofluidischen Analysesystems in geschnitten dargestellter Seitenansicht,
- Fig. 2:: das mikrofluidische Analysesystem gemäß Fig. 1 in geschnitten dargestellter Draufsicht,
- Fig. 3:: eine beispielhafte Veranschaulichung eines hier vorgeschlagenen Verfahrens,
- Fig. 4:: eine beispielhafte Trennpipette, die im Zusammenhang mit dem hier beschriebenen Analysesystem zur Anwendung kommen kann, und
- Fig. 5:: eine beispielhafte Veranschaulichung wie die Trennpipette aus Fig. 4 im Zusammenhang mit dem hier beschriebenen Analysesystem zur Anwendung kommen kann.

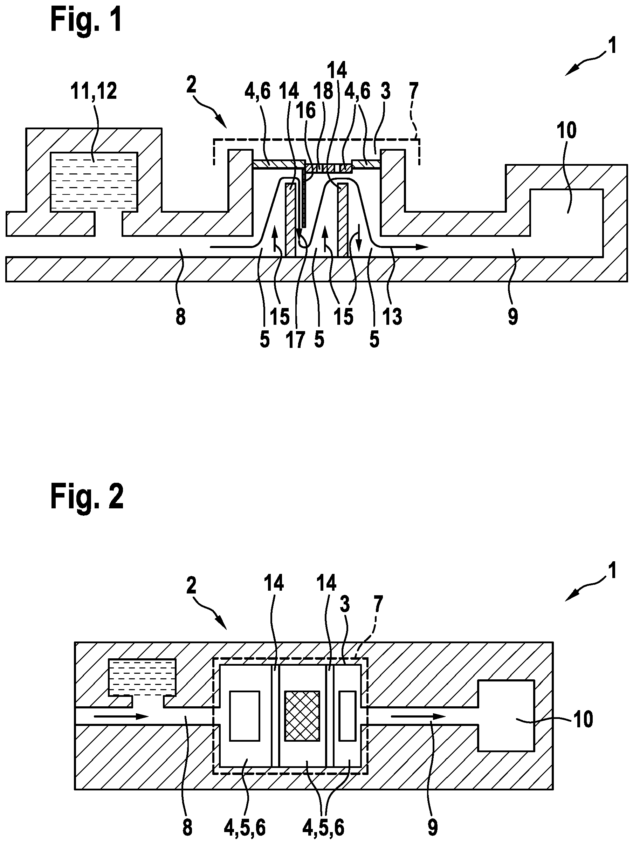

Fig. 1 zeigt schematisch eine beispielhafte Ausführungsform eines hier vorgeschlagenen mikrofluidischen Analysesystems 1 in geschnitten dargestellter Seitenansicht. Das mikrofluidische Analysesystem 1 ist zur Analyse von Blutproben eingerichtet. Es weist eine Probeneingabekammer 2 mit einer Öffnung 3 und drei räumlich voneinander getrennten Eingabebereichen 4 auf, in welche Bestandteile der Blutprobe getrennt voneinander eingebbar sind.

Weiterhin weisen die Eingabebereiche 4 jeweils einen Eingaberaum 5 und eine Zugangsöffnung 6 auf, wobei die Bestandteile der Blutprobe über die Zugangsöffnungen 6 in die Eingaberäume 5 der Eingabebereiche 4 eingebbar sind. Darüber hinaus existiert für die Probeneingabekammer 2 ein Deckel 7, mit welchem die Zugangsöffnungen 6 der Eingabebereiche 4 verschließbar sind.

Es ist auch gezeigt, dass die Probeneingabekammer 2 zwei Anschlussleitungen 8, 9 aufweist, über welche die Bestandteile der Blutprobe in Analysekammern 10 abtransportierbar sind und über welche die Probeneingabekammer 2 mit einem Förderfluid 11 beaufschlagbar ist. In diesem Zusammenhang weist das mikrofluidisches Analysesystem 1 ein Reservoir 12 auf, welches mit einer Förderfluid 11 gefüllt ist, das über eine erste Anschlussleitung 8 in die Probeneingabekammer 2 gefördert werden kann, um Bestandteile der Blutprobe aus der Probeneingabekammer 2 abzustransportieren.

In Fig. 1 ist auch veranschaulicht, dass die drei räumlich voneinander getrennten Eingabebereiche 4 entlang eines Strömungspfades 13 von einer ersten Anschlussleitung 8 an die Probeneingabekammer 2 zu einer zweiten Anschlussleitung 9 an die Probeneingabekammer 2 hintereinander angeordnet sind. Dabei sind die Eingabebereiche 4 durch zwei senkrecht ausgerichtete Trennwände 14 voneinander abgegrenzt.

Es ist in Fig. 1 zudem gezeigt, dass jeder Eingabebereich 4 entlang des Strömungspfades 13 eine Hauptdurchströmungsrichtung 15 von oben nach unten oder von unten nach oben hat. Zwei benachbart zueinander angeordnete Eingabebereiche 4 der Probeneingabekammer 2 (hier der mittlere und der rechte Eingabebereich 4) haben entlang des Strömungspfades 13 unterschiedliche Hauptdurchströmungsrichtungen 15. Zudem haben zwei benachbart zueinander angeordnete Eingabebereiche 4 der Probeneingabekammer 2 (hier der linke und der mittlere Eingabebereich 4) entlang des Strömungspfades 13 gleiche Hauptdurchströmungsrichtungen 15.

In diesem Zusammenhang ist in Fig. 1 auch gezeigt, dass zwischen den zwei benachbart zueinander angeordneten Eingabebereichen 4 mit gleicher Hauptdurchströmungsrichtung 15 eine Umlenkstrecke 16 existiert, mit welcher der Strömungspfad 13 in der zu der Hauptdurchströmungsrichtung 15 besagter Eingabebereiche 4 entgegengesetzten Richtung 17 umgelenkt ist.

In Fig. 1 ist darüber hinaus gezeigt, dass einer der Eingabebereiche 4 einen Eingabefilter 18 aufweisen kann, welcher den Eingabebereich 4 verdeckt.

Fig. 2 zeigt schematisch das mikrofluidische Analysesystem gemäß Fig. 1 in geschnitten dargestellter Draufsicht. Die Bezugszeichen werden einheitlich verwendet, sodass auf die vorhergehenden Erläuterungen zur Fig. 1 vollumfänglich Bezug genommen werden kann.

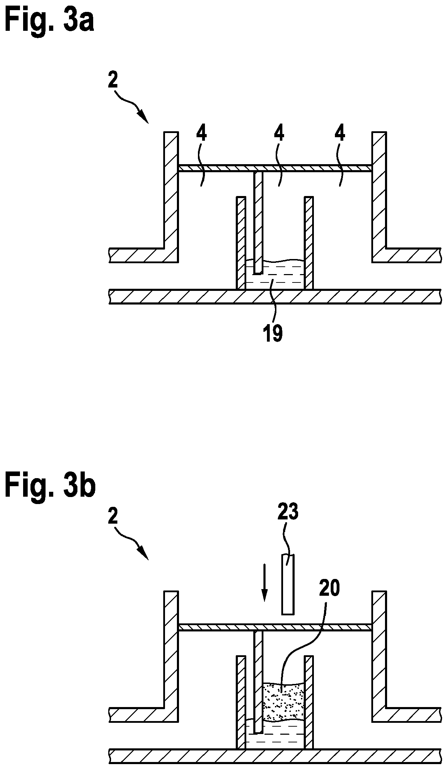

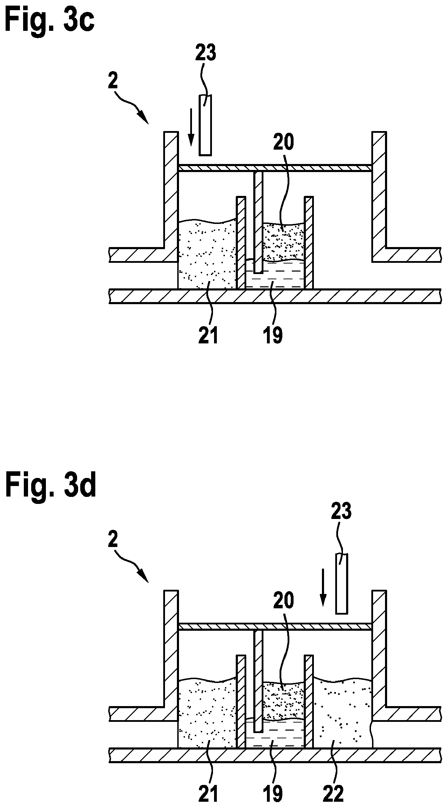

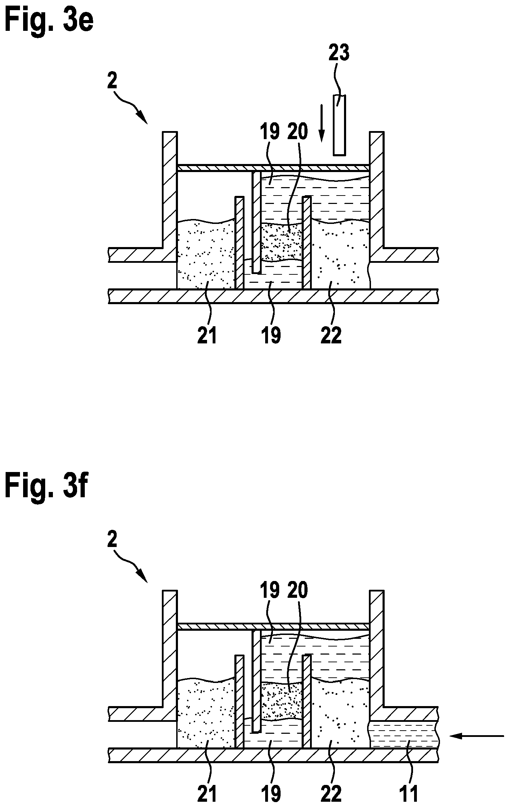

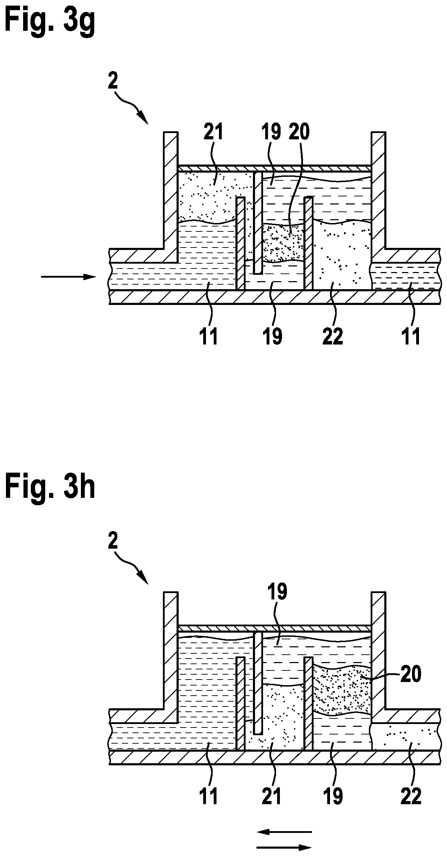

Fig. 3 zeigt schematisch eine beispielhafte Veranschaulichung eines hier vorgeschlagenen Verfahrens. Das Verfahren dient zum Eingeben von Bestandteilen einer Blutprobe 24 in ein hier beschriebenes mikrofluidisches Analysesystem 1 (vgl.

Fig. 1 und 2), das eine Probeneingabekammer 2 mit drei räumlich voneinander getrennten Eingabebereichen 4 aufweist.

Das Verfahren lässt sich im Wesentlichen in zwei Schritte gliedern. In einem ersten Schritt erfolgt eine zeitlich aufeinanderfolgende und voneinander getrennte Zugabe der Bestandteile der Blutprobe 24 in die Eingabebereiche 4 der Probeneingabekammer 2 gemäß einer Vorgabe. Dies ist beispielhaft in den Figuren 3a bis 3d veranschaulicht.

In einem zweiten Schritt erfolgt eine mindestens einmalige Zugabe eines Trennfluids 19 in die Eingabebereiche 4 der Probeneingabekammer 2 zur Abtrennung von Bestandteilen der Blutprobe 24 entlang des Strömungspfades 13. Dies ist beispielhaft in den Figuren 3e bis 3h veranschaulicht.

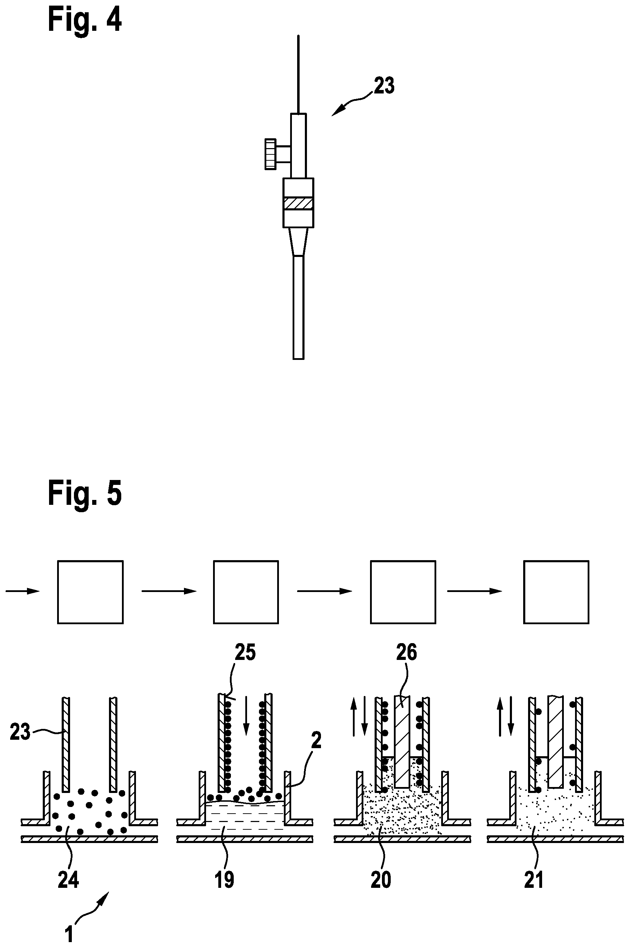

Bei den Bestandteilen der Blutprobe kann es sich beispielsweise um Extrakte einer Lyse handeln. Die Lyse bzw. Extraktion der Bestandteile kann zum Beispiel in einer hierzu eingerichteten Trennpipette 23 erfolgen, die in Fig. 3b angedeutet ist. Auf die Trennpipette 23 wird in den Figuren 4 und 5 näher eingegangen.

Um nun die Bestandteile der Blutprobe bzw. die Extrakte der Lyse, welche aus der Trennpipette 23 ausgestoßen werden, möglichst verlustfrei und/oder kontaminationsfrei in das mikrofluidische Analysesystem 1 überführen zu können, wird die beschriebene Probeneingabekammer 2 des Analysesystems 1 genutzt. Bei der Probeneingabekammer 2 kann es sich beispielsweise um eine sogenannte Polykammer handeln. Für diesen Prozess wird eine Probeneingabekammer 2 wie in Fig. 1 und Fig 2 gezeigt eingesetzt.

Die Probeeingabekammer 2, welche nach oben zur Atmosphäre offen ist und zwei Zugänge (µ-in durch Anschlussleitung 8; µ-out durch Anschlussleitung 9) zu einem mikrofluidschen System eines Lab-on-Chip hat, besteht dabei beispielhaft aus drei Teilkammern, welche die drei Eingabebereiche 4 bilden und welche nach oben miteinander verbunden sind. Um einen Durchfluss zu erzeugen, besitzt der Übergang von erster Kammer (linker Eingaberaum 5) zu zweiter Kammer (mittlerer Eingaberaum 5) einen sogenannten Flussguide (gebildet durch die Umlenkstrecke 16), welcher dazu beiträgt die überschwappende Lösung zu unterschichten (vgl. Fig. 3h).

Die erste Kammer (linker Eingaberaum 5) und die dritte Kemmer (rechter Eingaberaum 5), welche für die Cytosolextraktion respektive der Nukleusextraktion vorgesehen sind, sind nach oben geöffnet, sodass diese mit der Trennpipette 23 gefüllt werden können. Für das Abtrennen der Zellen vom Plasma besitzt die zweite Kammer (mittlerer Eingaberaum 5) einen Zellfilter (z.B. Nylonsieb mit Poren kleiner 2 µm) welche beispielsweise in einem Polykammeraufbauverfahren integriert aufgebaut werden können.

Fig. 3 demonstiert beispielhaft die mikrofluidische Sukzession zum verlustfreien und kontaminationsfreien Prozessieren der drei Extraktionen. Dazu ist beispielsweise Öl (oder ein anderes inertes, nicht-wasserlösliches Fluid) als Trennfluid 19 in der zweiten Kammer (mittlerer Eingaberaum 5) vorgelagert (vgl. Fig. 3a). Als erstes wird die Plasmafraktion als erster Bestandteil 20 der Blutprobe durch die Fluidmechanismen der Trennpipette 23 durch den Filter 18 gedrückt und in der zweiten Kammer bzw. dem mittleren Eingaberaum 5 zwischengelagert (Fig. 3b).

Im nächsten Schritt wird die Cytosolextraktion als zweiter Bestandteil 21 der Blutprobe in die erste Kammer bzw. den linken Eingaberaum 5 geben (vgl. Fig. 3c). Die letzte Extraktion, der Nukleusinhalt (dritter Bestandteil 22 der Blutprobe), wird dann in die letzte freie Kammer bzw. den rechten Eingaberaum 5 gegeben (vgl. Fig. 3d).

Um in einem letzten Schritt die Trennpipette 23 vollständig zu entleeren, kann durch diesen in der Regel Öl als Trennfluid 19 geschoben werden. Ein Teil oder die gesamte Menge davon kann mit in die Kammer bzw. einen oder mehrere der Eingaberäume 5 gegeben werden (vgl. Fig. 3e). Wird kein Öl bzw. Trennfluid 19 durch die Trennpipette 23 geschoben, kann ein kleine Menge Öl bzw. Trennfluid 19 noch zusätzlich vom Nutzer über beispielsweise die erste Kammer bzw. den mittleren Eingaberaum 5 gegeben werden.

Nun befinden sich alle drei Extraktionen bzw. alle drei Bestandteile 20, 21, 22 der Blutprobe 24 von Öl und Luft getrennt in der Kammer. Dies kann auch so beschrieben werden, dass alle drei Bestandteile 20, 21, 22 der Blutprobe 24 durch das Trennfluid 19 (welches z. B. Öl und Luft umfassen kann) voneinander getrennt in der Probeneingabekammer 2 vorgehalten sind.

Das z.B. in der Art eines Lab-on-Chip gebildete Analysesystem 2 kann nun in einen hier nicht dargestellten Analysierer (z B. eine Analyseeinrichtung bzw. ein Analysegerät) eingegeben werden und automatisch fertig prozessiert werden. Mit anderen Worten können die voneinander getrennten Bestandteile 20, 21, 22 nun in ein mit der Probeneingabekammer 2 verbundenes Analysegerät (hier nicht dargestellt) transportiert werden.

Dieser Transport erfolgt üblicherweise über eine mikrofluidische Förderung des Trennfluids 19, welches die Bestandteile 20, 21, 22 einschließt. Alternativ oder kumulativ können das Trennfluid 19 und die Bestandteile 20, 21, 22 zur Förderung mit einem zusätzlichen Förderfluid 11 umgeben werden. Dies wird allgemein auch als Transport im Zweiphasensystem bezeichnet.

Dabei wird zuerst der erste Zugangskanal (Anschlussleitung 9) mit Öl als Trennfluid 19 und/oder Förderfluid 11 befüllt und somit die Luft aus dem Kanal entfernt (vgl. Fig. 3f). Dies geschieht insbesondere bevor derselbe fluidische Schritt mit dem zweiten Zugangskanal (Anschlussleitung 8) gemacht wird (vgl. Fig. 3g). Durch die Zugabe von Öl als Trennfluid 19 und/oder Förderfluid 11 durch die Zugangskanäle 8, 9, verbinden sich die Kammer bzw. Eingaberäume 5 zu einer gemeinsamen Kammer und es kann ein Durchfluss gemäß dem Polykammernprinzip erzeugt werden.

Bevor nun die Probe im Zweiphasensystem nach dem Prinzip des verlustfreien Sample-Einzugs ausgetragen wird, können noch mögliche Restluftblasen durch ein Hin- und Herbewegen innerhalb der Probeneingabekammer 2 (vgl. Fig. 3h) aus den Fluiden entfernt werden und somit eine blasenfreien, kontaminationsfreien und verlustfreien Probeneinzug und Verteilung auf dem Lab-on-Chip ermöglichen.

Fig. 4 zeigt schematisch eine beispielhafte Trennpipette 23, die im Zusammenhang mit dem hier beschriebenen Analysesystem 1 zur Anwendung kommen kann. Die Trennpipette 23 bildet die Hardware, die zur Durchführung der im Zusammenhang mit Fig. 5 beschriebenen Schritte eingesetzt wird. Diese wird insbesondere dafür verwendet, um eine Blutprobe in die genannten drei Bestandteile 20, 21, 22 zu zerlegen, um diese drei Bestandteile 20, 21, 22 dann anschließend getrennt voneinander in den hier beschriebenen Zugabeport bzw. die Probeneingabekammer 2 eines mikrofluidischen Analysesystems 1 einzubringen.

Fig. 5 zeigt schematisch eine beispielhafte Veranschaulichung wie die Trennpipette 23 aus Fig. 4 im Zusammenhang mit dem hier beschriebenen Analysesystem zur Anwendung kommen kann. Fig. 5 demonstriert in diesem Zusammenhang die anzuwenden fluidischen Schritte, welche auf die Zellen angewandt werden.

In einem ersten Schritt werden Zellen der Blutprobe 24 fixiert (linkes Bild in Fig. 5). Dazu werden diese in eine Kapillare der Trennpipette 23 eingezogen, welche mit einem für Blutzellen adhärenten Material beschichtet wurde, um eine Haftfläche 25 zu bilden. Dabei sättigt sich der Innenmantel der Kapillare mit der immer gleichen Mengen an Zellen.

Die in der Kapillare nicht haftenden Zellen werden nicht wieder ins Gefäß der ursprünglichen Blutprobe 24 gegeben, sondern in ein Gefäß, welches mit einem Zellfilter ausgestattet ist (vgl. zweites Bild von links in Fig. 5). Bei letzterem Gefäß kann es sich beispielsweise um eine Probeneingabekammer 2 eines mikrofluidischen Analysesystems 1 (insbesondere um den in Fig. 3 mittleren Eingabebereich 4 der oben erläuterten Probeneingabekammer 2) handeln. Somit findet eine Extraktion des Plasmas (erster Bestandteil 20) statt, da die restlichen Zellen auf diesem Filter (z.B. Eingabefilter 18) durch ihre Größe zurückgehalten werden und insbesondere das Blutplasma mit seinen Bestandteilen (Proteine und zellfreie-DANN) separiert wurde.

In einem nächsten Schritt (zweites Bild von rechts in Fig. 5) findet die Extraktion des Cytosols (zweiter Bestandteil 21) statt. Dabei werden die in der Kanüle haftenden Zellen selektiv lysiert. Um dies in einem kleinen Volumen zu ermöglichen, wird ein zentrierter Zylinder als Verdrängerstab 26 eingeschoben, welcher das Totalvolumen reduziert. Unter selektiver Lyse versteht man hier beispielsweise einen Lysepuffer, welcher nur die Zellmembran durchlässig macht, nicht aber den Zellkern. Dabei bleiben Zytoskelett inklusive dem geschützten Nukleus immobilisiert, während die Proteine und RNA des Zytosols extrahiert und in ein neues Gefäß (z.B. den in Fig. 3 linken Eingabebereich 4 der oben beschriebenen Probeneingabekammer 2) ausgestoßen werden können. Als Lysepuffer dienen dazu hypotone Lösungen wie ein Phosphatpuffer mit einem NaCl Anteil über 150 mM und Triton versetzt.

Als Letztes folgt dann (rechtes Bild in Fig. 5) die Extraktion des Nukleus (dritter Bestandteil 22), insbesondere der darin enthaltene DNA. Dazu wird ein hypertonsicher Lysepuffer eingezogen, z. B. destilliertes Wasser. Dabei platzt der Zellkern auf und die DNA wird extrahiert. Dieses Extrakt wird auch im verkleinerten Volumen der Kapillare gewonnen und in ein neues Gefäß (insbesondere den in Fig. 3 rechten Eingabebereich 4 der oben beschriebenen Probeneingabekammer 2) transferiert.

Diese drei fluidischen Schritte erlauben in vorteilhafter Weise das Trennen der drei Komponenten, Plasma (erster Bestandteil 20), Cytosol (zweiter Bestandteil 21) und Nukleus (dritter Bestandteil 22). Insbesondere kann daraus zellfreie DNA, (transkriptorische) RNA und genomische DNA von einer defnierten Menge Blutzellen aus einer Probe und selben Zellpopulation am Point-of-care getrennt werden.

Mit dem angegebenen Analysesystem und/oder dem Verfahren können insbesondere einer oder mehrere der folgenden Vorteile erreicht werden:
- Der mikrofluidische Prozess ermöglicht eine normierte Blutprobenaufbereitung für Multiparametermessungen. Durch den angewandten Prozess wird im Durchschnitt immer die gleiche Anzahl an Blutzellen in die nachfolgende Analyse überführt. Dies ist für die Vergleichbarkeit und Quantifizietung eine wichtige Qualitätsbedingung und erlaubt ein quantitatives Patienten-Monitoring.
- Es können mehrere Größen aus derselben Probe, ohne große Verluste, gemessen werden. Insbesondere können ohne Effizienzeinbußen DNA und RNA von denselben Zellen gemessen werden.
- Es kann eine definierte Anzahl von Zellen von einem großen in ein kleines Volumen überführt werden und somit die Konzentration der Analyten gesteigert werden.
- Es können drei verschiedene Analyten kontaminationsfrei von einer Ausgangsprobe in ein Lab-on-Chip eingezogen werden.
- Das Verfahren besteht aus vier händischen Fluidschritten, welche ohne große Mühe von patientennahem Personal (Ärzte, Pfleger, MPA) ausgeführt werden können und keine Analyselaborfähigkeiten benötigen. Daher eignet sich das Verfahren ideal als Point-of-care Verfahren.
- Die Methode stellt eine universelle Probeaufbereitung für Blut dar und ist von Targets unabhängig.

## Patentansprüche

1. Mikrofluidisches Analysesystem (1) zur Analyse von Blutproben, aufweisend eine Probeneingabekammer (2) mit einer Öffnung (3) und drei räumlich voneinander getrennten Eingabebereichen (4), in welche Bestandteile der Blutprobe getrennt voneinander eingebbar sind, **dadurch gekennzeichnet, dass** die Probeneingabekammer (2) mindestens zwei Anschlussleitungen (8,9) aufweist, über welche die Bestandteile der Blutprobe in Analysekammern (10) abtransportierbar sind und über welche die Probeneingabekammer (2) mit einem Förderfluid (11) beaufschlagbar ist, ferner aufweisend mindestens ein Reservoir (12), welches mit einer Förderfluid (11) gefüllt ist, die über eine erste Anschlussleitung (8) in die Probeneingabekammer (2) gefördert werden kann, um Bestandteile der Blutprobe aus der Probeneingabekammer (2) abzustransportieren.

2. Mikrofluidisches Analysesystem (1) nach Anspruch 1, wobei die Eingabebereiche (4) jeweils einen Eingaberaum (5) und eine Zugangsöffnung (6) aufweisen, wobei die Bestandteile der Blutprobe über die Zugangsöffnungen (6) in die Eingaberäume (5) der Eingabebereiche (4) eingebbar sind.

3. Mikrofluidisches Analysesystem (1) nach Anspruch 2, wobei für die Probeneingabekammer (2) ein Deckel (7) existiert, mit welchem die Zugangsöffnungen (6) der Eingabebereiche (4) verschließbar sind.

4. Mikrofluidisches Analysesystem (1) nach einem der vorhergehenden Ansprüche, wobei die drei räumlich voneinander getrennten Eingabebereiche (4) entlang eines Strömungspfades (13) von einer ersten Anschlussleitung (8) an die Probeneingabekammer (2) zu einer zweiten Anschlussleitung (9) an die Probeneingabekammer (2) hintereinander angeordnet sind.

5. Mikrofluidisches Analysesysteme (1) nach einem der vorhergehenden Ansprüche, wobei die Eingabebereiche (4) durch mindestens zwei senkrecht ausgerichtete Trennwände (14) voneinander abgegrenzt sind.

6. Mikrofluidisches Analysesystem (1) nach Anspruch 4, wobei jeder Eingabebereich (4) entlang eines Strömungspfades (13) eine Hauptdurchströmungsrichtung (15) von oben nach unten oder von unten nach oben hat, wobei zwei benachbart zueinander angeordnete Eingabebereiche (4) der Probeneingabekammer (2), entlang des Strömungspfades (13) jeweils unterschiedliche Hauptdurchströmungsrichtungen (15) haben.

7. Mikrofluidisches Analysesystem (1) nach Anspruch 4, wobei jeder Eingabebereich (4) entlang eines Strömungspfades (13) eine Hauptdurchströmungsrichtung (15) von oben nach unten oder von unten nach oben hat, wobei zwei benachbart zueinander angeordnete Eingabebereiche der Probeneingabekammer, entlang des Strömungspfades (13) gleiche Hauptdurchströmungsrichtungen (15) haben.

8. Mikrofluidisches Analysesystem (1) nach Anspruch 7, wobei zwischen zwei benachbart zueinander angeordneten Eingabebereichen (4) mit gleicher Hauptdurchströmungsrichtung (15) eine Umlenkstrecke (16) existiert mit welcher der Strömungspfad (13) in der zu der Hauptdurchströmungsrichtung (15) besagter Eingabebereiche (4) entgegengesetzten Richtung (17) umgelenkt ist.

9. Mikrofluidisches Analysesystem (1) nach einem der vorhergehenden Ansprüche, wobei mindestens einer der Eingabebereiche (4) einen Eingabefilter (18) aufweist, welcher den Eingabebereich (4) verdeckt.

10. Verfahren zum Eingeben von Bestandteilen einer Blutprobe in ein mikrofluidisches Analysesystem (1) nach einem der vorhergehenden Ansprüche aufweisend die folgenden Schritte:
a) Zeitlich aufeinanderfolgende und voneinander getrennte Zugabe der Bestandteile der Blutprobe in die Eingabebereiche (4) der Probeneingabekammer (2), gemäß einer Vorgabe,
b) Mindestens einmalige Zugabe eines Trennfluids (19) in die Eingabebereiche (4) der Probeneingabekammer (2) zur Abtrennung von Bestandteilen der Blutprobe entlang des Strömungspfades (13) von einer ersten Anschlussleitung (8) an die Probeneingabekammer (2) zu einer zweiten Anschlussleitung (9) an die Probeneingabekammer (2).

## Claims

1. Microfluidic analysis system (1) for analysing blood samples, having a sample input chamber (2) with an opening (3) and with three spatially separated input regions (4) into which constituents of the blood sample can be input separately from one another, **characterized in that** the sample input chamber (2) has at least two connection lines (8, 9) via which the constituents of the blood sample can be transported away into analysis chambers (10) and via which a conveying fluid (11) can be supplied to the sample input chamber (2), moreover having at least one reservoir (12) which is filled with a conveying fluid (11) which can be conveyed via a first connection line (8) into the sample input chamber (2) in order to transport constituents of the blood sample away from the sample input chamber (2).

2. Microfluidic analysis system (1) according to Claim 1, wherein the input regions (4) each have an input space (5) and an access opening (6), wherein the constituents of the blood sample can be introduced into the input spaces (5) of the input regions (4) via the access openings (6).

3. Microfluidic analysis system (1) according to Claim 2, wherein for the sample input chamber (2) there is a lid (7), with which the access openings (6) of the input regions (4) can be closed.

4. Microfluidic analysis system (1) according to one of the preceding claims, wherein the three spatially separated input regions (4) are arranged one after another along a flow path (13) from a first connection line (8) to the sample input chamber (2) to a second connection line (9) to the sample input chamber (2).

5. Microfluidic analysis system (1) according to one of the preceding claims, wherein the input regions (4) are delimited from one another by at least two vertically oriented partition walls (14).

6. Microfluidic analysis system (1) according to Claim 4, wherein each input region (4) along a flow path (13) has a main flow direction (15) from the top downwards or from the bottom upwards, wherein two mutually adjacent input regions (4) of the sample input chamber (2) each have different main flow directions (15) along the flow path (13).

7. Microfluidic analysis system (1) according to Claim 4, wherein each input region (4) along a flow path (13) has a main flow direction (15) from the top downwards or from the bottom upwards, wherein two mutually adjacent input regions of the sample input chamber have the same main flow directions (15) along the flow path (13).

8. Microfluidic analysis system (1) according to Claim 7, wherein, between two mutually adjacent input regions (4) with the same main flow direction (15), there is a deflection path (16) with which the flow path (13) is deflected in the direction (17) counter to the main flow direction (15) of said input regions (4).

9. Microfluidic analysis system (1) according to one of the preceding claims, wherein at least one of the input regions (4) has an input filter (18), which covers the input region (4).

10. Method for introducing constituents of a blood sample into a microfluidic analysis system (1) according to one of the preceding claims, having the following steps:
a) Successive and separate addition of the constituents of the blood sample into the input regions (4) of the sample input chamber (2), according to a specification,
b) At least one-off addition of a separating fluid (19) into the input regions (4) of the sample input chamber (2) for the separation of constituents of the blood sample along the flow path (13) from a first connection line (8) to the sample input chamber (2) to a second connection line (9) to the sample input chamber (2).

## Revendications

1. Système d'analyse microfluidique (1) pour l'analyse d'échantillons de sang, présentant une chambre d'introduction d'échantillon (2) avec une ouverture (3) et trois zones d'introduction (4) séparées spatialement les unes des autres, dans lesquelles des constituants de l'échantillon de sang peuvent être introduits séparément les uns des autres, **caractérisé en ce que** la chambre d'introduction d'échantillon (2) présente au moins deux conduites de raccordement (8,9), par le biais desquelles les constituants de l'échantillon de sang peuvent être évacués dans des chambres d'analyse (10) et par le biais desquelles la chambre d'introduction d'échantillon (2) peut être sollicitée avec un fluide de transport (11), présentant en outre au moins un réservoir (12) qui est rempli d'un fluide de transport (11) qui peut être transporté dans la chambre d'introduction d'échantillon (2) par le biais d'une première conduite de raccordement (8) afin d'évacuer des constituants de l'échantillon de sang de la chambre d'introduction d'échantillon (2).

2. Système d'analyse microfluidique (1) selon la revendication 1, dans lequel les zones d'introduction (4) présentent chacune un espace d'introduction (5) et une ouverture d'accès (6), les constituants de l'échantillon de sang pouvant être introduits dans les espaces d'introduction (5) des zones d'introduction (4) par le biais des ouvertures d'accès (6).

3. Système d'analyse microfluidique (1) selon la revendication 2, dans lequel il existe un couvercle (7) pour la chambre d'introduction d'échantillon (2), avec lequel les ouvertures d'accès (6) des zones d'introduction (4) peuvent être fermées.

4. Système d'analyse microfluidique (1) selon l'une quelconque des revendications précédentes, dans lequel les trois zones d'introduction (4) séparées spatialement les unes des autres sont agencées les unes derrière les autres le long d'un chemin d'écoulement (13) allant d'une première conduite de raccordement (8) à la chambre d'introduction d'échantillon (2) jusqu'à une deuxième conduite de raccordement (9) à la chambre d'introduction d'échantillon (2).

5. Système d'analyse microfluidique (1) selon l'une quelconque des revendications précédentes, dans lequel les zones d'introduction (4) sont délimitées les unes par rapport aux autres par au moins deux parois de séparation (14) orientées verticalement.

6. Système d'analyse microfluidique (1) selon la revendication 4, dans lequel chaque zone d'introduction (4) a une direction d'écoulement principale (15) de haut en bas ou de bas en haut le long d'un chemin d'écoulement (13), dans lequel deux zones d'introduction (4) de la chambre d'introduction d'échantillon (2) agencées de manière voisine l'une de l'autre le long du chemin d'écoulement (13) ont respectivement des directions d'écoulement principales différentes (15).

7. Système d'analyse microfluidique (1) selon la revendication 4, dans lequel chaque zone d'introduction (4) a une direction d'écoulement principale (15) de haut en bas ou de bas en haut le long d'un chemin d'écoulement (13), dans lequel deux zones d'introduction de la chambre d'introduction d'échantillon agencées de manière voisine l'une de l'autre ont des directions d'écoulement principales (15) identiques le long du chemin d'écoulement (13).

8. Système d'analyse microfluidique (1) selon la revendication 7, dans lequel il existe, entre deux zones d'introduction (4) agencées de manière voisine l'une de l'autre avec une direction principale d'écoulement (15) identique, une section de déviation (16) avec laquelle le chemin d'écoulement (13) est dévié dans la direction (17) opposée à la direction principale d'écoulement (15) desdites zones d'introduction (4).

9. Système d'analyse microfluidique (1) selon l'une quelconque des revendications précédentes, dans lequel au moins une des zones d'introduction (4) présente un filtre d'introduction (18) qui masque la zone d'introduction (4).

10. Procédé d'introduction de constituants d'un échantillon de sang dans un système d'analyse microfluidique (1) selon l'une quelconque des revendications précédentes, présentant les étapes suivantes :
a) l'ajout successif et séparé dans le temps des constituants de l'échantillon de sang dans les zones d'introduction (4) de la chambre d'introduction d'échantillon (2), conformément à une consigne,
b) l'ajout au moins une fois d'un fluide de séparation (19) dans les zones d'introduction (4) de la chambre d'introduction d'échantillon (2) pour séparer les constituants de l'échantillon de sang le long du chemin d'écoulement (13) d'une première conduite de raccordement (8) à la chambre d'introduction d'échantillon (2) jusqu'à une deuxième conduite de raccordement (9) à la chambre d'introduction d'échantillon (2).
